# EUROPEAN PATENT SPECIFICATION

(11) **EP 1 242 307 B1**
(45) Date of publication and mention of the grant of the patent: **27.04.2005**
(21) Application number: 00990794.0
(22) Date of filing: 18.12.2000
(51) Int. Cl.: C01B 33/12

(54) **METHOD FOR PREPARING ORTHO SILICIC ACID, ORTHO SILICIC ACID AS OBTAINED, AND ITS USE**
VERFAHREN ZUR HERSTELLUNG VON ORTHOKIESELSÄURE, SOWIE NACH DEM VERFAHREN HERGESTELLTE KIESELSÄURE UND IHRE VERWENDUNG
PROCEDE DE PREPARATION D'ACIDE ORTHOSILICIQUE, ACIDE ORTHOSILICIQUE AINSI OBTENU, ET SON UTILISATION

(30) Priority: 24.12.1999 EP 99204496
(43) Date of publication of application: 25.09.2002
(73) Proprietor: Bio Minerals N.V., 9070 Destelbergen (BE)
(72) Inventor: VANDEN BERGHE, Dirk, André, Richard, B-9270 Laarne (BE)
(74) Representative: Prins, Hendrik Willem
(86) International application number: PCT/EP2000/012929
(87) International publication number: WO 2001/047807

(56) References cited:
- WO-A-95/21124
- HERBERT FUNK: "Über Lösungen der Monokieselsäure Si(OH)4" CHEMISCHES ZENTRALBLATT, no. 4, 1964, page 47 XP002138346
- DATABASE WPI Section Ch, Week 199215 Derwent Publications Ltd., London, GB; Class C04, AN 1992-118232 XP002138349 & JP 04 059614 A (YG AKIYAMA), 26 February 1992 (1992-02-26)
- CHEMICAL ABSTRACTS, vol. 127, no. 7, 18 August 1997 (1997-08-18) Columbus, Ohio, US; abstract no. 99539, WASHIO YUTAKA: "SURFACE-TREATED INORGANIC POWDERS FOR COSMETICS" XP002138347 & JP 09 143032 A (NARISU COSMETIC CO) 3 June 1997 (1997-06-03)
- CHEMICAL ABSTRACTS, vol. 86, no. 24, 13 June 1977 (1977-06-13) Columbus, Ohio, US; abstract no. 173652, SLINYAKOVA I. B. ET AL.: "Hygroscopic film adsorbent based on silicic acid" XP002138348 & SU 540 654 A 30 December 1976 (1976-12-30)

## Description

The present invention relates to a method for preparing ortho silicic acid, to the ortho silicic acid obtainable by this method and to its use as a silicone preparation in the production of animal feed, food, food or feed supplement, and for the production of a pharmaceutical or cosmetic preparation. Silicon (Si) has been recognized as an essential trace element for diatoms, Si accumulating plants and higher animals. The best documented function of silicon in vertebrates is its regulatory action in bone calcification and its chemical association with several constituents of the extracellular matrix in connective tissues (Carlisle E. (1989), Silicon, in : *Handbook of Nutritionally Essential Mineral Elements,* ed. B.L. O'Dell and R.A. Sunde, Marcel Dekker Inc., New York, pp. 603-618). This matrix consists primarily of fibrous proteins such as collagen, embedded in a hydrated polysaccharide gel. Silicon being bound to components of this matrix is regarded to be important for the structural integrity, the development and the regulatory functions of connective tissue. Gastro-intestinal absorption of Si is only possible after hydrolysis of dietary Si-compounds into ortho silicic acid. The solubility of silicon compounds in the diet is low and consequently these compounds have a limited bioavailability. Organic compounds comprising Si-C bounds are not found in biological systems and several classes of synthetized products were found to have an unacceptable high toxicity. The natural soluble silicon compound, ortho silicic acid also called monomeric silicic acid is present both in fresh and sea water but only at very low concentrations (<1 mmol l⁻¹ [Sullivan C. (1986) Silicification by diatoms, in *: Silicon* *Biochemistry, CIBA Foundation Symposium 121*, John Wiley and Sons, New York, pp. 24-39].) Higher concentrations in aqueous media initiates a polymerization reaction of into non-bioavailable colloids and ultimately gels. A method for the preparation of a stabilized formulation of ortho silicic acid is disclosed US 5,922,360.

The present invention has for its object to provide a method for preparing ortho silicic acid starting from relatively inexpensive and market available starting materials while polymerisation of formed ortho silicic acid is substantially avoided.

This is obtained with the method according to the invention for preparing ortho silicic acid wherein an acid hydrolysable silicon compound is hydrolysed in an acid solution in the presence of a non toxic solvent agent under the formation of ortho silicic acid, such as a acid aqueous solution. Due to the use of an acid solution and to the presence of a non toxic solvent agent the afore mentioned polymerisation reaction is substantially suppressed and the ortho silicic acid formed is sufficiently stabilized.

The starting material, which is an acid hydrolizable silicon compound, may be selected from a silicate, such as a monomeric silicate such as silicon halogenide, methyl ortho silicate, sodium or magnesium orthosilicates, or from hydrated silicate such as crystalline sodium silicate.

According to another embodiment the acid hydrolizable silicon compound has the general formula 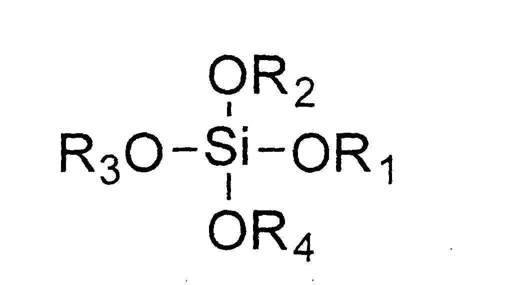 wherein R₁, R₂, R₃ and R₄ are independently selected from H, C₁-C₁₂ alkyl, C₁-C₁₂ alkoxy which are optionally substituted by an hydroxyl group, under the proviso that R₁, R₂, R₃ and R₄ are not simultaneously H. Preferably, R₁, R₂, R₃ and R₄ are selected from H, C₁-C₄ alkyl, C₁-C₄ alkoxy optionally substituted by an hydroxylgroup. It is noted that R₁, R₂, R₃ and R₄ are preferably selected such that the compound split off from the hydrolisable silicon compound is removable using traditional techniques such as evaporation and distillation, and most preferably is non-toxic (LD₅₀ orally in rat higher than lg/kg bodyweight). The most preferred silicon compound is tetra-ethoxy-silanol.

Other preferred examples for R are C₂H₅, CH₃CO, HCO, C₃H₇, C₄H₉ and CH₃CH(OH)CHCO. The solution may comprise 1-80%, preferably 10-70%, more preferably 40-60% solvent agent.

The non toxic solvent agent used in the acid solution for stabilizing the formed ortho silicic acid may be selected from the group comprising glycol, glycerol, (poly)alkylene glycol, DMSO and polysorbate 80. The (poly) alkylene glycol may be polypropylene glycol or polyethylene glycol. The alkylene glycol may be ethylene glycol or propylene glycol. A common set of properties for all non toxic solvent agents are a high solubility in water (more than 30%), a boiling point higher than 130°C, a liquid state between -10°C and 40°C and a stability at an acid pH of generally 0-4.

The formed ortho silicic acid stabilized by the non toxic solvent agent, may be stabilized further by contacting the ortho silicic acid with a non toxic particulate carrier.

Surprisingly, it is experienced that this non toxic particulate carrier adsorbed ortho silicic acid has a bioavailability which is comparable or even improved over the stabilized formulation, as disclosed in US 5,922,360. The bioavailability is a critical issue since it was recently shown in comparative human supplementation studies that solid silicon supplements such as colloidal silica and phytolytic silicates are not bioavailable whereas a solution of stabilized ORTHO SILICIC ACID in a HCl-choline matrix has a high bioavailability [Calomme M., Cos P., Vingerhoets R., Van Hoorebeke C., Vanden Berghe D. (1998) Comparative bioavailability study of silicon supplements in healthy subjects, *Journal of Parenteral and Enteral Nutrition,* 22, S12, (abstract #47) .Van Dyck K., Van Cauwenbergh R., Robberecht H., Deelstra H. (1999), Bioavailability of silicon from food and food supplements, *Fresenius Journal of Analytical Chemistry,* 363, 541-544.] Accordingly, the present invention also provides a silicon preparation, comprising ortho silicic acid adsorbed on a particulate carrier, obtainable by the process comprising the steps of:
i) providing a solution, comprising ortho silicic acid stabilized with said acid solvent agent; and
ii) contacting the ortho silicic acid comprising solution with the particulate carrier.
In order to avoid to an additional extent the polymerization of ortho silicic acid, it is preferred that the ortho silicic acid is formed in situ. The handling and the formation of dosing forms of the silicon preparation are further improved when the carrier, after contact with ortho silicic acid, is extruded.

The skilled person will appreciate that the silicon preparation according to the invention may contain ortho silicic acid over a broad silicon content range depending on the contemplated use of the silicon preparation. Generally, the silicon content of the silicon preparation is within the range of 0.01-50 wt.%, preferably within the range of 0.01-10 wt.%, more preferably within the range of 0.1-10 wt.%, and most preferably within the range of 0.1-5 wt.%. Accordingly, the silicon preparation may be used in a dosing regime which is suitable for most contemplated food, feed, pharmaceutical and cosmetic utilities. In this respect it is noted that the pharmaceutical and cosmetic preparation will have a positive effect on nails, hair, skin, teeth, collagen, connetive tissue, bones, encourages cell generation, stimulates the immune system against infections and toxins and inhibits degenerative (ageing)-process. In addition it is noted that the solvent agent and carrier should be non toxic which means not initiating adverse toxic effects in man, animal and plant.

Experimental use of silicon preparations according to the invention have shown, that the silicon preparation has a desired high bioavailability expressed as the total silicon absorption by an organism such as a human being. Over a period of 0-8 hours the relative bioavailability was much improved over the afore mentioned colloidal and phytolytic silica preparations. In other words the total silicon absorption over 8 hours is more than 250 µg Si.h/l, preferably more than 500 µg Si.h/l, more preferably more than 600 µg Si.h/l, such as 250-700 µg Si.h/l, preferably 300-700 µg Si.h/l.

The silicon preparation according to the invention adsorbed on a carrier may be used as such or in combination with any acceptable carrier material, excipient or diluent.

The silicon preparation according to the invention may be administared orally or in any other suitable fashion. Oral administration is preferred and the silicon preparation may have the form of a tablet, aqueous dispersion, dispersable powder or granule, emulsion, hard or soft capsule, syrup, elixir or gel. The dosing forms may be prepared using any method known in the art for manufacturing these pharmaceutical or cosmetic compositions and may comprise as additives sweeteners, flavoring agents, coloring agents, preservatives and the like.. Carrier materials and excipients may include calcium carbonate, sodium carbonate, lactose, calcium phosphate or sodium phosphate; granulating and disintegrating agents, binding agents and the like. The silicon preparation may be included in a gelatin capsule mixed with any inert solid diluent or carrier material, or has the form of a soft gelatin capsule, in which the ingredient is mixed with a water or oil medium. Aqueous dispersions may comprise the silicon preparation in combination with a suspending agent, dispersing agent or wetting agent. Oil dispersions may comprise suspending agents such as a vegetable oil. A gel formulation may be prepared following the teaching given in US 5,922,360.

It is now possible to make dry mixtures of carrier-bound ortho silicic acid with other components such as trace elements, vitamins, amino acids, sugars, plant extracts, and other ingredients used in the manufacturing of food and food supplements. As an explanation it is considered that the ortho silicic acid remains in its monomeric form in carrier-bound ortho silicic acid and is therefore different from non-bioavailable polymerized forms of ortho silicic acid such as in colloidal or solid silicic acid and silicates.
Ortho silicic acid is for instance prepared in the presence of the acid solvent agent and in situ by (a) hydrolysis of monomeric silicon compounds such as silicon halogenide or methyl orchosilicate [Iler R. (1979) Monosilicic acid, in : The Chemistry of Silica, John Wiley and Sons, New York, pp. 178-180.), (b) by reacting monomeric silicates such as sodium or magnesium orthosilicates or hydrated crystalline sodium silicate with dilute acid (Iler 1979), (c) by hydolyzing organic alkylsilanol compounds. It is noted that next to the formed ortho silicic acid the other hydrolization reaction compounds should be non-toxic and if desired should be removed from the reaction mixture. Preferably, the alkylsilanol compound is an ethoxysilanol compound and the formed ethanol may be separated without difficulty. The freshly prepared ortho silicic acid is bound to the carrier or a combination of carriers. A second method is to bind first a organic silicon compound on a carrier and thereafter hydrolyzing the organic silicon compound into ortho silicic acid for instance at a pH of lower than 4, such as 0.2-2.5, more preferably 0.8-1.0.

The solid carrier or combination of solid carriers may be selected from the group comprising:
i) natural and semi-synthetic fibers,
ii) plant metabolites such as polyfenols, lignans, flavonoid,
iii) fatty acids and esters thereof such as stearates, palmitates, linoleates, oleates, adipates, caprylates, caprates, cocoates,
iv) phosholipids and derivates thereof,
v) polyalcohols such as inositol, trehalose,
vi) hydrogenated and sulfated compounds,
vii) salts such as chlorides, sulfates, nitrates, etc.,
viii) pectines and alginates,
ix) sugars or sugar alcohols and derivatives thereof such as lactose, sucrose, mannitol, sorbitol, sorbitolesters,
x) poly- and oligosaccharides silicic acharides and derivatives thereof such as dextran, fructans, inulin, oligofructose,
xi) gelatine or derivatives thereof such as gelatine hydrolysate
xii) cellulose er derivatives thereof such as microcrystalline cellulose,hydroxypropylcellulose, hydroxypropyl methylcellulose,carboxymethylcellulose, cellulose gum
xiii) peptides and polypeptides such as collagen, soy proteins, mays protein and derivates thereof
xiv) glucans and derivatives thereof such as proteoglycans, glycosaminoglycans, hyaluronic acid, chondroitin sulfate, heparin, heparan sulfate, keratan sulfate, dermatan sulfate,
xv) starch and derivatives thereof,
xvi) lecithin and derivatives thereof, and
xvii) byproducts of foodproduction, such as fermented byproducts from cheese, beer and mays, and cheese whey as an example
xviii) foodproducts such as dried animal food, substrates for plants such as natural peat for plant production, dried plant extracts are dried plant homogenates and cosmetic powders such as talc.

### Example A

Ortho silicic acid is prepared as followed. Two liters of a fresh solution of cold sodiumsilicate (27 % SiO₂ in 14 % NaOH) is mixed with 2 - 4 liters of glycerol (pro analyse, 100 %) until a homogeneous solution is obtained. To decrease the pH, one liter of cold, concentrated hydrochloric acid is added and the mixture is stirred strongly at a temperature between 0 - 10 °C. During continuous mixing, solid or a suspension of calciumcarbonate is added until a pH of 1-3 is obtained. During mixing CO₂ gas will be formed.
Half a liter of freshly prepared combination of concentrated ortho silicic acid is mixed with 0.5 kg of gelatine, or 0.5 kg of cheese whey, or 200 g of cellulose, or 1 kg of galactose, or 1 kg of saccharose. The resulting paste is mixed until a homogeneous paste is obtained. The paste is dried in vacuo. The final product contains minimum 0.1 % elemental silicon and preferably between 1 - 5 % elemental silicon.
A daily intake of 0.5 g during 2 months resulted in improved nail and hair quality in four different persons. This improvement was equivalent as observed using the formulations mentioned in US 5,922,360.

### Example B

The carrier (65 %) microcrystalline cellulose is mixed with 35 % of a combination of concentrated ortho silicic acid with glycerol (see example A). Demineralized water is added during continuous mixing to obtain an appropiate quality of the granulated material. The plastic mass is extruded with a basket extruder (Caleva Model 10, Sturminster Newton, Great Britain) at 750 rpm. The extruded strands are spheronized (Caleva Model 120 spheronizer). The resulting pellets are dried to a final water content of lower than 5 %. Typical pellet size is between 800 and 1200 µm. The pellets are encapsulated in hard gelatine capsules size OO. Each capsule contains 0.54 g pellets equal to 5 mg elemental silicon in the form of carrier-bound ortho silicic acid. The loading capacity of the microcrystalline cellulose can be increased to 45 % ortho silicic acid.

### Example C

The carrier, a mixture (1:1) of soy proteins and mays proteins (70 %) are mixed with 30 % of a combination of ortho silicic acid with glycerol (see example A). Demineralized water is added during continuous mixing to obtain an homogenous plastic mass. The mixture is dried by lyophilization. Following granulation the protein-bound ortho silicic acid is directly encapsulated or used as a raw material in the manufacturing of animal feed, food, food supplements, cosmetics or pharmaceutical preparations.

### Example D

The carrier (65 %) a mixture (3:1) of microcrystalline cellulose and fructans is mixed with 35 % of a combination of concentrated ortho silicic acid with glycerol (see example A). Demineralized water is added during continuous mixing to obtain an appropiate quality of the granulated material. The plastic mass is extruded with a basket extruder (Caleva Model 10, Sturminster Newton, Great Britain) at 750 rpm. The extruded strands are spheronized (Caleva Model 120 spheronizer). The resulting pellets are dried to a final water content of lower than 5 %. Typical pellet size is between 800 and 1400 µm. The pellets are pressed to tablets or used as a raw material in the manufacturing of animal feed, food, food supplements, cosmetics or pharmaceutical preparations.

### Example E

100 ml icecold tetra-ethoxy-silanol is dropped slowly in 1 liter of 50% solution icecold glycerol in water pH 1,0. After 8 h at 0°C the silanol compound is completely hydrolysed. Ethanol is removed by quick evaporation under vacuum. The remaining OSA solution is mixed with 2 - 3 kg lactose as a paste and further dried under vacuum. The final product contains minimum 0.1 % Si and preferably between 0.3 abd 2% Si.

Dissolution assays of the preparations of Examples A-E prove that ortho silicic acid is released within 30 minutes into the dissolution medium. This is demonstrated by measuring the silicon content of the dissolution medium at fixed time-points with Zeeman corrected Electrothermal Atomic Absorption Spectometry (Perkin Elmer). The fact that ortho silicic acid is released during dissolution demonstrates clearly that binding of ortho silicic acid to the carrier will not result in polymerization of ortho silicic acid but remains in a dissociatable form. Dissolution assays were repeated at 3, 6 and 12 months after the production date without difference in results demonstrating that carrier-bound ortho silicic acid is chemically stable over a long period of time.

### Example F

Three healthy subjects (2 females, 1 male, aged 22-34 y) were included after informed, written consent. None had taken Si supplements within 3 months before the start of the study. Each fasting subject received in a cross-over protocol Si p.o. as follows:10 mg of Si in the form of stabilized ortho silicic acid (ortho silicic acid, 0.5 ml of BioSil containing 20 g Si/l, as in US 5, 922,360), 10 mg of Si in the form of carrier-bound ortho silicic acid (capsules of the preparation of Example D), 20 mg of Si in the form of colloidal silica (polymerized ortho silicic acid) 20 mg of Si in the form of phytolytic silica (a standarized dry extract of the Si-accumulating plant Equisetum arvense) or a placebo (10 ml mineral water) within 1 week washout period between each supplement or the placebo. Blood samples were collected in Si free polypropylene tubes prior to supplementation and after 1, 2, 4, 6 and 8 hours post partem. Identical meals were consumed during the experiment after 2 and 6 hours supplementation. The Si concentration in serum and urine was determined for each subject in one batch with AAS. A Zeeman/3030 Atomic Absorption Spectrometer equipped with a HGA-600 graphite furnace was used in combination with an AS-60 autosampler (Perkin-Elmer Corp. Norwalk CT). The area under the time concentration curve (A.U.C.) was calculated using the linear trapezoidal rule as an objective parameter of the total Si absorption. The serum silicon concentration increases significantly from the baseline value after supplementation of both liquid ortho silicic acid and carrier-bound ortho silicic acid (fig. 1 ortho silic acid = OSA) but not after supplementation of polymerized ortho silicic acid forms such as colloidal silica or phytolytic silica. The kinetic absorption profile for carrier-bound ortho silicic acid indicates a slower-release effect compared to liquid ortho silicic acid. The total bioavailability is similar for carrier-bound ortho silicic acid and liquid ortho silicic acid whereas the polymerized forms of ortho silicic acid are not bioavailable since no significant difference is seen for these products compared to the placebo (fig. 2 ortho silicic acid = OSA). Bioavailability experiments were repeated one year after the production date of the carrier -bound orthosilicic acid without significant differences in results, demonstrating that carrier-bound orthosilicic acid is chemically stable over a long period of time without significant loss in bioavailability. Figure 3 illustrates the kinetic profile in serum for volunteers (n=3) supplemented with 10 mg silicon in the form of (a) OSA liquid, (b) fresh prepared carrier-bound OSA, and (c) 1 year old carrier-bound OSA . The relative bioavailability calculated as the area under the time curve (A.U.C.) was not significantly different between the different silicon forms (mean ± SD): 132 ± 28 µg h/L for placebo, 795 ± 231 µg h/L for OSA liquid, 869 ± 448 µg h/L for fresh prepared carrier-bound OSA, and 622 ± 251 µg h/L for 1 year old carrier-bound OSA respectively.

### Example G

Feed-pellets for sows ('the carrier') are mixed with a combination of concentrated ortho silicic acid with glycerol (see example A) until a concentration of 15 mg Si/ kg feed in the form of carrier-bound OSA is obtained. Sows are fed daily 4kg of this 'carrier-bound OSA' diet starting 1 week before insemination until weaning. A control group of sows received a normal feed, identical in composition except for the presence of carrier-bound OSA. Blood was withdrawn from the piglets at the age of 4 weeks (weaning) and the silicon concentration was determined in the serum with graphite furnace atomic absorption spectrometry. The mean serum silicon concentration in piglets fed the 'carrier-bound OSA' diet was 150 % higher compared to the controls (table 1) which illustrates clearly that (a) carrier-bound OSA has a high bioavailability, (b) the absorbed silicon from carrier-bound OSA is tranferred between the lactating sow and the offspring by either the placenta or the milk or a combination of both.

**Table 1 :**

| The effect of feeding sows a OSA-bound diet on the serum silicon concentration of the offspring. | |
|---|---|
| Dietary group of sows | Si concentration in serum of offspring (piglets) mean ± SE (ppb) |
| Regular control diet | 109 ± 8 |
| | |
| OSA-bound diet | 277 ± 20 |

Fig. 1 Increase in silicon concentration in serum from the baseline value in healthy subjects after supplementation of respectively 10 mg Si in the form of carrier-bound OSA, 10 mg Si in the form of liquid OSA, 20 mg Si in the form of colloidal silica, 20 mg of Si in the form of phytolytic silica.

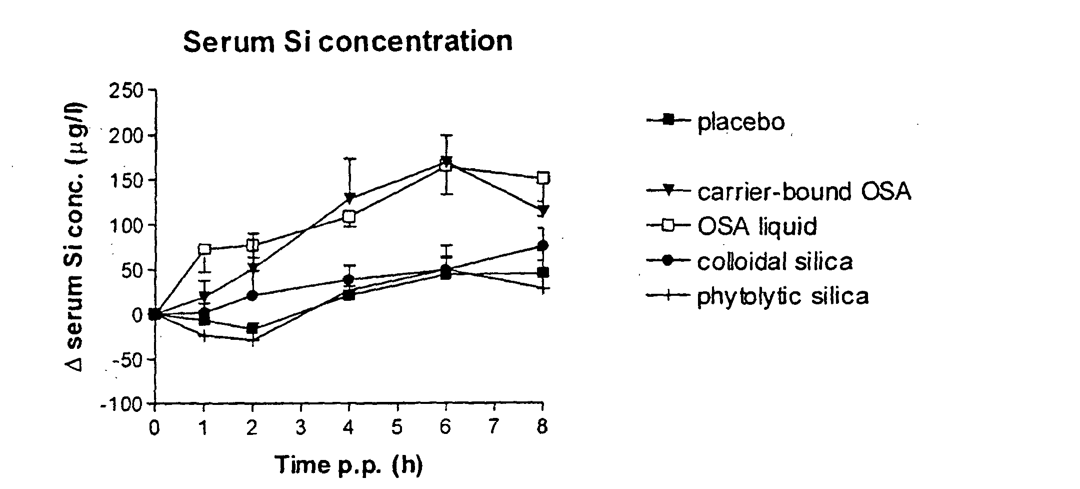

Fig. 2 Total absorption of silicon in serum over a period of 0-8 hours p.p. measured in healthy subjects after supplementation of respectively 10 mg Si in the form of carrier-bound OSA, 10 mg Si in the form of liquid OSA, 20 mg Si in the form of colloidal silica, 20 mg of Si in the form of phytolytic silica.

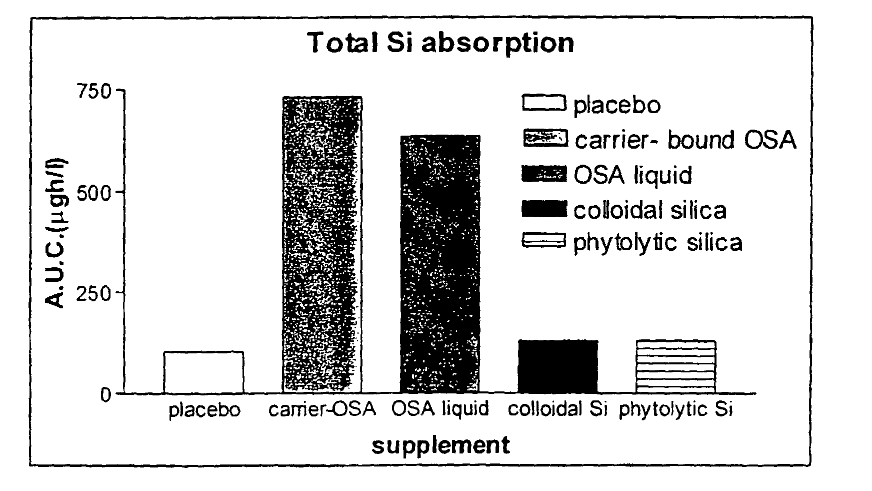

**Fig. 3** Effect of one year shelf life of carrier-bound OSA on the increase in serum silicon concentration in healthy subjects.

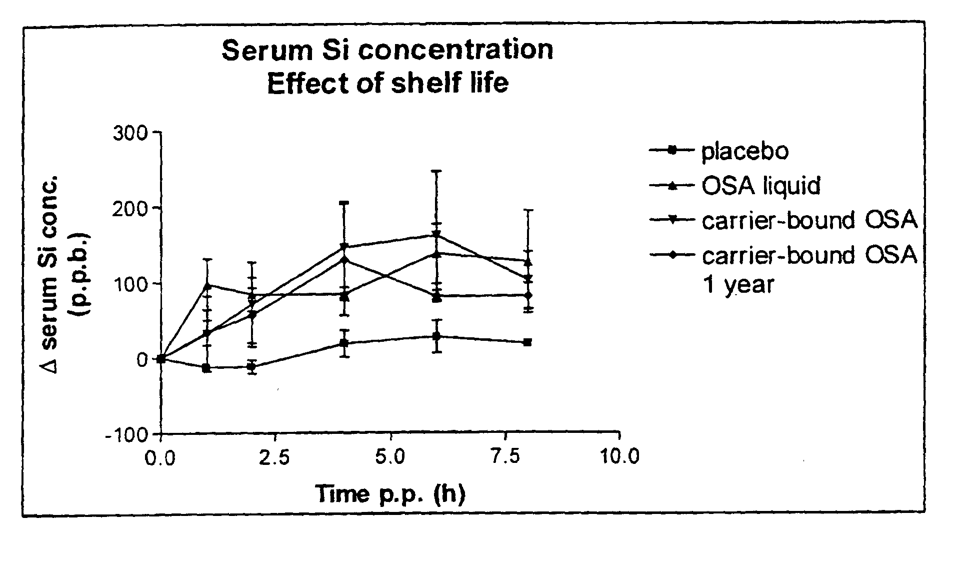

## Claims

1. Method for preparing ortho silicic acid wherein an acid hydrolysable silicon compound is hydrolysed in an acid aqueous solution having a pH of 0-4, in the presence of a non toxic solvent agent having a boiling point higher than 130°C, a liquid state between -10°C and 40°C and stable at a pH of generally 0-4, under the formation of ortho silicic acid.

2. Method as claimed in claim 1, wherein the acid hydrolysable silicon compound is a silicate, such as a monomeric silicate or hydrated silicate.

3. Method as claimed in claim 1, wherein the acid hydrolysable silicon compound has the general formula 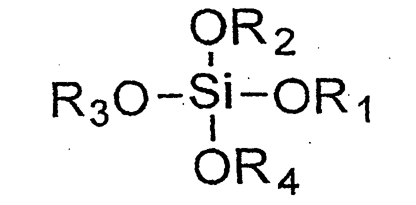 wherein R₁, R₂, R₃ and R₄ are independently selected from H, C₁-C₁₂, alkyl, C₁-C₁₂ which are alkoxy optionally substituted by an hydroxyl group, under the proviso that R₁, R₂, R₃ and R₄ are not simultaneously H.

4. Method as claimed in claim 3, wherein R₁, R₂, R₃ and R₄ are selected from H, C₁-C₄ alkyl, C₁-C₄ alkoxy optionally substituted by (an) hydroxylgroup (s).

5. Method as claimed in claims 1-4, wherein the non-toxic solvent agent is selected from: glycol, glycerol, (poly) alkylene glycol, DMSO and polysorbate 80.

6. Method as claimed in claims 1-5, wherein the solution comprises 1-80%, preferably 10-70%, more preferably 40-60% solvent agent.

7. Method as claimed in claims 1-6, wherein the acid solution has a pH of 0.2-2.5, preferably 0.8-1.0.

8. Method as claimed in claims 1-7, wherein the ortho silicic acid formed is contacted with a non toxic particulate carrier.

9. Method as claimed in claim 8, wherein the ortho silicic acid is formed in situ in the presence of the particulate carrier.

10. Method as claimed in claim 8 or 9, wherein the carrier, after contact with ortho silicic acid, is extruded.

11. Method as claimed in claims 8-10, wherein the silicon preparation has a silicon content of 0.01-50 wt.%, preferably 0.01-10 wt.%, more preferably 0.1-10 wt.%, most preferably 0.1-5 wt.%.

12. Method as claimed in claims 8-11, wherein the silicon preparation has a total silicon absorption over 8 hours of more than 250 (µg) Si.h/l, preferably more than 500 (µg) Si.h/l, more preferably more than 600 (µg) Si.h/l, such as 250-700 (µg) Si.h/l, preferably 300-700 (µg) Si.h/l.

13. Use of a silicon preparation as formed in claims 1-7 or produced in claims 8-12, in the production of animal feed, food or feed supplement, and of a pharmaceutical or cosmetic preparation.

14. Ortho silicic acid based preparation comprising ortho silicic acid and a non toxic solvent agent obtainable by the method of claims 1-7.

## Patentansprüche

1. Verfahren zur Herstellung von Orthokieselsäure, bei welchem eine säure-hydrolysierbare Siliciumverbindung in einer sauren wässrigen Lösung mit einem pH-Wert von 0-4 in Gegenwart eines nicht toxischen Lösungsmittels mit einem Siedepunkt größer als 130 °C, einem flüssigen Zustand zwischen -10 °C und 40 °C und stabil bei einem pH-Wert von allgemein 0-4, unter Bildung von Orthokieselsäure hydrolysiert wird.

2. Verfahren gemäß Anspruch 1, bei welchem die säurehydrolysierbare Siliciumverbindung ein Silikat ist, wie etwa ein monomeres Silikat oder ein hydratisiertes Silikat.

3. Verfahren gemäß Anspruch 1, wobei die säurehydrolysierbare Siliciumverbindung die allgemeine Formel 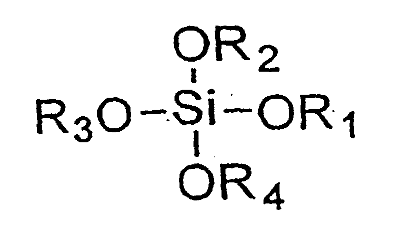 aufweist, wobei R₁, R₂, R₃ und R₄ unabhängig ausgewählt sind aus H, C₁-C₁₂, Alkyl, C₁-C₁₂, deren Alkoxy optional durch eine Hydroxylgruppe substituiert sind, unter der Voraussetzung, dass R₁, R₂, R₃ und R₄ nicht gleichzeitig H sind.

4. Verfahren gemäß Anspruch 3, wobei R₁, R₂, R₃ und R₄ ausgewählt sind aus H, C₁-C₄ Alkyl, C₁-C₄ Alkoxy, optional substituiert durch (eine) Hydroxylgruppe(n).

5. Verfahren gemäß Ansprüchen 1-4, wobei das nicht toxische Lösungsmittel ausgewählt ist aus: Glycol, Glycerol, (Poly)Alkylenglycol, DMSO und Polysorbat 80.

6. Verfahren gemäß Ansprüchen 1-5, wobei die Lösung 1-80 %, bevorzugt 10-70 %, besonders bevorzugt 40-60 % Lösungsmittel umfasst.

7. Verfahren gemäß Ansprüchen 1-6, wobei die saure Lösung einen pH von 0,2-2,5, bevorzugt 0,8-1,0 aufweist.

8. Verfahren gemäß Ansprüchen 1-7, bei welchem die gebildete Orthokieselsäure mit einem nicht toxischen partikelförmigen Träger kontaktiert wird.

9. Verfahren gemäß Anspruch 8, bei welchem die Orthokieselsäure in situ in Gegenwart des partikelförmigen Trägers gebildet wird.

10. Verfahren gemäß Anspruch 8 oder 9, bei welchem der Träger nach Kontakt mit Orthokieselsäure extrudiert wird.

11. Verfahren gemäß Ansprüchen 8-10, bei welchem die Siliciumzubereitung einen Siliciumgehalt von 0,01-50 Gew.-%, bevorzugt 0,01-10 Gew.-%, besonders bevorzugt 0,1-10 Gew.-%, am bevorzugtesten 0,1-5 Gew.-% aufweist.

12. Verfahren gemäß Ansprüchen 8-11, bei welchem die Silicuimzubereitung eine Gesamtsiliciumabsorption über 8 Stunden von mehr als 250 (µg) Si.h/l, bevorzugt mehr als 500 (µg) Si.h/l, besonders bevorzugt mehr als 600 (µg) Si.h/l, wie etwa 250-700 (µg) Si.h/l, bevorzugt 300-700 (µg) Si.h/l, aufweist.

13. Verwendung einer Siliciumzubereitung, wie sie in den Ansprüchen 1-7 gebildet oder in den Ansprüchen 8-12 hergestellt wird, bei der Herstellung von Tiernahrung, Lebensmitteln oder Nahrungsergänzungsmitteln und von einer pharmazeutischen oder kosmetischen Zubereitung.

14. Auf Orthokieselsäure basierende Zubereitung, welche Orthokieselsäure und ein nicht toxisches Lösungsmittel umfasst und durch das Verfahren der Ansprüche 1-7 erhältlich ist.

## Revendications

1. Procédé de préparation d'acide ortho-silicique, dans lequel un composé de silicium hydrolysable par un acide est hydrolysé dans une solution aqueuse acide ayant un pH de 0 à 4, en présence d'un agent solvant non toxique ayant un point d'ébullition supérieur à 130°C, un état liquide compris entre -10°C et 40°C et stable à un pH généralement de 0 à 4, dans les conditions de formation de l'acide ortho-silicique.

2. Procédé selon la revendication 1, dans lequel le composé de silicium hydrolysable par un acide est un silicate, tel qu'un silicate monomère ou un silicate hydraté.

3. Procédé selon la revendication 1, dans lequel le composé de silicium hydrolysable par un acide répond à la formule générale 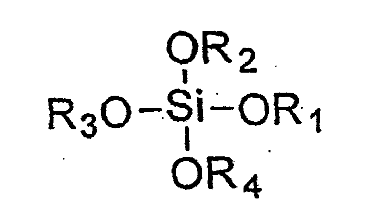 dans laquelle R₁, R₂, R₃ et R₄ sont choisis indépendamment parmi H, alkyle en C₁-C₁₂, alcoxy en C₁-C₁₂ qui sont éventuellement substitués par un groupe hydroxyle, à condition que R₁, R₂, R₃ et R₄ ne soient pas simultanément H.

4. Procédé selon la revendication 3, dans lequel R₁, R₂, R₃ et R₄ sont choisis parmi H, alkyle en C₁-C₄, alcoxy en C₁-C₄ éventuellement substitués par un ou des groupes hydroxyle.

5. Procédé selon l'une quelconque des revendications 1 à 4, dans lequel l'agent solvant non toxique est choisi parmi : le glycol, le glycérol, un (poly)alkylèneglycol, le DMSO et le polysorbate 80.

6. Procédé selon l'une quelconque des revendications 1 à 5, dans lequel la solution comprend 1 à 80 %, de préférence 10 à 70 %, mieux encore 40 à 60 %, d'agent solvant.

7. Procédé selon l'une quelconque des revendications 1 à 6, dans lequel la solution acide a un pH de 0,2 à 2,5, de préférence de 0,8 à 1,0.

8. Procédé selon l'une quelconque des revendications 1 à 7, dans lequel l'acide ortho-silicique formé est mis en contact avec un support particulaire non toxique.

9. Procédé selon la revendication 8, dans lequel l'acide ortho-silicique est formé in situ en présence du support particulaire.

10. Procédé selon la revendication 8 ou 9, dans lequel le support, après la mise en contact avec l'acide ortho-silicique, est extrudé.

11. Procédé selon l'une quelconque des revendications 8 à 10, dans lequel la préparation à base de silicium a une teneur en silicium de 0,01 à 50 % en poids, de préférence de 0,01 à 10 % en poids, mieux encore de 0,1 à 10 % en poids, tout particulièrement de 0,1 à 5 % en poids.

12. Procédé selon l'une quelconque des revendications 8 à 11, dans lequel la préparation à base de silicium a une absorption totale de silicium en l'espace de 8 heures supérieure à 250 (µg) de Si.h/l, de préférence supérieure à 500 (µg) de Si.h/l, mieux encore supérieure à 600 (µg) de Si.h/l, telle que de 250 à 700 (µg) de Si.h/l, de préférence de 300 à 700 (µg) de Si.h/l.

13. Utilisation d'une préparation à base de silicium formée dans les revendications 1 à 7 ou produite dans les revendications 8 à 12, dans la production d'une alimentation animale, d'aliment ou d'un complément alimentaire, et d'une préparation pharmaceutique ou cosmétique.

14. Préparation à base d'acide ortho-silicique comprenant de l'acide ortho-silicique et un agent solvant non toxique, susceptible d'être obtenue par le procédé des revendications 1 à 7.
